# EUROPÄISCHE PATENTSCHRIFT

(11) **EP 1 620 023 B1**
(45) Veröffentlichungstag und Bekanntmachung des Hinweises auf die Patenterteilung: **05.11.2008**
(21) Anmeldenummer: 04715265.7
(22) Anmeldetag: 27.02.2004
(51) Int. Cl.: A61B 17/70

(54) **DYNAMISCHE VERANKERUNGSVORRICHTUNG UND DYNAMISCHE STABILISIERUNGSEINRICHTUNG FÜR KNOCHEN, INSBESONDERE FÜR WIRBEL, MIT EINER DERARTIGEN VERANKERUNGSVORRICHTUNG**
DYNAMIC ANCHORING DEVICE AND DYNAMIC STABILIZING DEVICE, WHICH SERVES TO STABILIZE BONES, PARTICULARLY VERTEBRAE, AND WHICH HAS AN ANCHORING DEVICE OF THIS TYPE
DISPOSITIF D'ANCRAGE DYNAMIQUE, ET DISPOSITIF DE STABILISATION DYNAMIQUE CON U POUR DES OS, EN PARTICULIER LES VERTEBRES, COMPORTANT LEDIT DISPOSITIF D'ANCRAGE

(30) Priorität: 07.05.2003 DE 10320417
(43) Veröffentlichungstag der Anmeldung: 01.02.2006
(73) Patentinhaber: BIEDERMANN MOTECH GmbH, 78054 VS-Schwenningen (DE)
(72) Erfinder: HARMS, Jürgen, 76227 Karlsruhe (DE); BIEDERMANN, Lutz, 78048 VS-Villingen (DE); RAPP, Helmar, 78652 Deisslingen (DE)
(74) Vertreter: Hofer, Dorothea
(86) Internationale Anmeldenummer: PCT/EP2004/001978
(87) Internationale Veröffentlichungsnummer: WO 2004/098423

(56) Entgegenhaltungen:
- US-A- 5 672 175
- US-A- 5 961 517
- US-A- 6 022 350
- US-A- 6 113 601
- US-A1- 2001 034 521
- US-B1- 6 355 040

## Beschreibung

Die Erfindung betrifft eine dynamische Verankerungsvorrichtung und eine dynamische Stabilisierungseinrichtung für Knochen, insbesondere für Wirbel, mit einer derartigen Verankerungsvorrichtung.

Eine bekannte Methode der Behandlung von Bandscheibendefekten ist die operative Entnahme der defekten Bandscheibe und die knöcherne Versteifung des Bandscheibenzwischenraums mit den beiden benachbarten Wirbelkörpern. Bei dieser Methode werden die an das versteifte Wirbelsäulensegmentangrenzenden Abschnitte der Wirbelsäulevor allem im Bereich der Bandscheibe überlastet. Eine andere Behandlungsmethode besteht darin, die defekte Bandscheibe zu entnehmen und anschließend eine künstliche Bandscheibe einzusetzen. Da in den überwiegenden Fällen auch die hinteren Facettengelenke und der Bandapparat stark geschädigt sind, ist eine natürliche Bewegungskontrolle der künstlichen Bandscheibe von der posterioren Seite her in der Regel nicht mehr gegeben. Damit wirken hohe Scher- und Drehkräfte verschleißend auf das behandelnde Wirbelsäulensegment ein.

Aus der DE 42 39 715 C2 ist ein Fixationssystem zur Stabilisierung von wirbeln- bekannt, bei dem sich Rotations- und Druck/zug--Wechselbelastungen auf das im Knochen fixierte Teil vermindern lassen. Eine elastische Dämpfung oder Bewegungsführung der auftretenden Bewegungen ist jedoch nicht möglich.

Aus der EP 0 669 109 81 ist eine Vorrichtung zum Stabilisieren von benachbarten Rückenwirbeln bekannt mit der eine geschädigte Bandscheibe und die Zwischenwirbelgelenke posterior teilentlastet werden können. Die Vorrichtung weist zwei Pedikelschrauben auf, die mit einem aus einem elastischen Kunststoff bestehenden Band jeweils fest verbunden sind und über das vorgespannte Band miteinander verbunden sind. Zur Übertragung von Druckkräften ist ferner ein auf das elastische Band aufgeschobener druckfester Körper zwischen den zwei Schraubenköpfen vorgesehen. Die Verwendung eines solchen Textilbandes mit einem druckfesten Körper ermöglicht jedoch keine mehrachsige Führungsstabilität des Bewegungssegments einer Wirbelsäule.

Aus der US 5,474,555 ist eine Polyaxial-Knochenschraube mit einem Schraubenelement und einem mit diesem verbundenen Aufnahmeteil für einen Stab bekannt, die eine begrenzte Dewegung zwischen dem Aufnahmeteil und dem Wirbel erlaubt. Eine elastische Dämpfung der Bewegung ist mit dieser Schraube jedoch nicht möglich.

Aus der US 5,716,356 ist ein verankerungselement in Form einer Polyaxial-KnochensGhraube bekannt, bei dem das auf den Kopf einwirkende Druckelement ein Federelement beinhaltet, das auf den eingelegten Stab wirkt.

Aus der US 6,022,350 ist eine Knochchenfixationsvorrichtung in Form einer Platte mit Öffnungen, durch die sich Knochenschrauben hindurch erstrecken, bekannt. Die Knochenschrauben können in verschiedenen Winkelstellungen orientiert sein. Dic Kno chenschrauben sind mittels einer in die Öffnung einschraubbaren Schraube fixierbar. Zum Dämpfen der Steifigkeit der Vorrichtung ist ein Kügelchen oder ein O-Ring aus einem dämpfenden Material vorgesehen, das bzw. der beim Anziehen der in die Öffnung einschraubbaren Schraube komprimiert und deformiert wird.

US 5,961,517 offenbart ein Verankerungselement mit einer Knochenschraube und mit einem Aufnahmeteil mit einer U-förmigen Ausnehmung zum Einlegen eines Stabs und einer Fixierschraube zum Fixieren des Stabs in der Ausnehmung, sowie mit einem auf dem Kopf der Knochenschraube einwirkenden Druckelement. Das Druckelement weist eine U-förmige Ausnehmung zum Aufnehmen des Stabs auf und eine Feder auf dem Grund dieser Ausnehmung. Wenn die Fixierschraube festgezogen ist und der Stab fixiert ist, ist die gesamte Verbindung zwischen Schraube und Aufnahmeteils und Stab starr und erlaubt keine Bewegung der Schraube relativ zum Aufnahmeteil.

US 6 113 601 offenbart eine Verankerungsvorrichtung mit den Merkmalen des Oberbegriffs des Anspruchs 17.

Es ist Aufgabe der Erfindung, eine Verankerungsvorrichtung vorzusehen, die eine dynamische Verankerung einer mechanischen Vorrichtung, wie z.B. eines Stabs in einem Knochen oder einem Wirbel erlaubt und die insbesondere für eine dynamische Stabilisierungseinrichtung zur stabilisierenden Bewegungsführung und Entlastung einer künstlichen Bandscheibe einsetzbar ist. Ferner ist es Aufgabe der Erfindung eine dynamische Stabilisierungseinrichtung für Knochen, insbesondere für Wirbel bereitzustellen, die eine Bewegungskontrolle und Entlastung der menschlichen Bandscheibe von der posterioren Seite her erlaubt.

Die Aufgabe wird gelöst durch eine Verankerungsvorrichtung nach Anspruch 1 bzw. nach Anspruch 17. Weiterbildungen der Erfindung sind in den Unteransprüchen angegeben.

Weitere Merkmale der Erfindung ergeben sich aus der Beschreibung von Ausführungsbeispielen anhand der Figuren.

Von den Figuren zeigen:
- Fig. 1: eine teilgeschnittene Darstellung einer ersten Ausführungsform der Verankerungsvorrichtung im unbelasteten Zustand;
- Fig. 2: eine teilgeschnittene Darstellung der Verankerungsvorrichtung nach Fig. 1 im belasteten Zustand in Ruhestellung;
- Fig. 3: eine teilgeschnittene Darstellung der Verankerungsvorrichtung von Fig. 1 im belasteten Zustand bei Einwirkung einer Kraft auf das Verankerungselement;
- Fig. 4: eine schematische Darstellung einer zweiten Ausführungsform des Druckelements der in den Fig. 1 bis 3 gezeigten Ausführungsform des Verankerungselements;
- Fig. 5a: eine teilgeschnittene Darstellung einer zweiten Ausführungsform des Verankerungselements;
- Fig. 5b-5e: Herstellungsschritte zur Herstellung des Schraubenelements einer Abwandlung der zweiten Ausführungsform;
- Fig. 6: eine Schnittdarstellung einer dritten Ausführungsform des Verankerungselements;
- Fig. 7: eine schematische Darstellung der ersten Ausführungsform der dynamischen Stabilisierungseinrichtung;
- Fig. 8: eine schematische Darstellung einer zweiten Ausführungsform der dynamischen Stabilisierungseinrichtung in einem ersten Anwendungsfall;
- Fig. 9: eine schematische Darstellung der zweiten Ausführungsform der dynamischen Stabilisierungseinrichtung in einem zweiten Anwendungsfall;
- Fig. 10a-c: eine Schnittdarstellung einer weiteren Ausführungsform der dynamischen Verankerungssvorrichtung; und
- Fig. 11: eine Schnittdarstellung einer weiteren Ausführungsform der dynamischen Verankerungsvorrichtung im unbelasteten Zustand;
- Fig. 12: die in Fig. 11 dargestellte Ausführungsform im belasteten Zustand; und
- Fig. 13: eine schematische Darstellung der Funktionsweisen der in Fig. 11 und 12 dargestellten Ausführungsform.

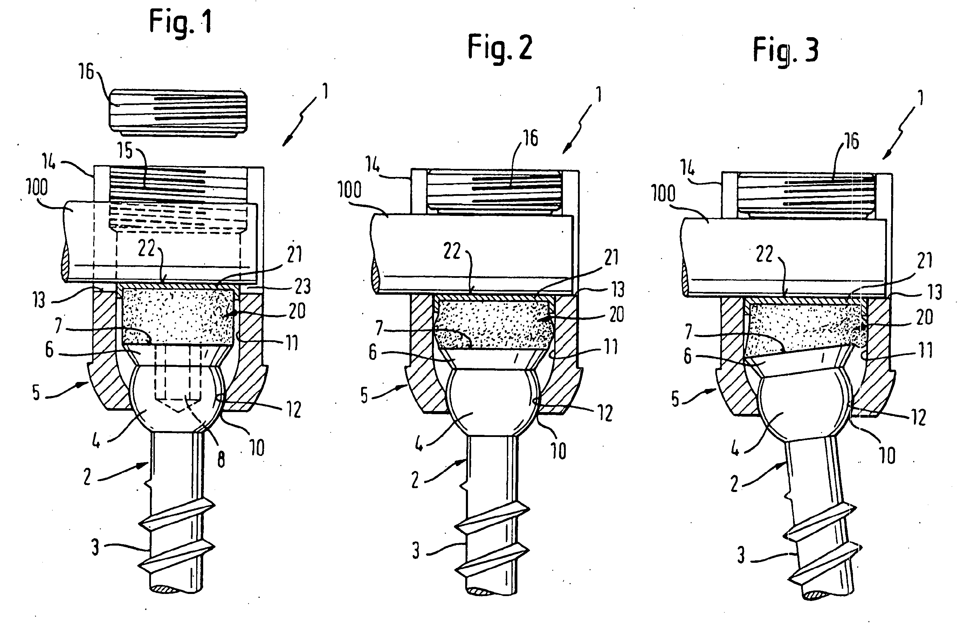

Wie insbesondere aus den Fig. 1 bis 3 ersichtlich, ist das dynamische Verankerungselement 1 als Polyaxialschraube ausgebildet. Diese weist ein Schraubenelement 2 mit einem Gewindeschaftteil 3 und einem einstückig damit ausgebildetem Kopf 4 sowie ein Aufnahmeteil 5 auf. Der Kopf 4 ist im wesentlichen kugelsegmentförmig ausgebildet und weist an seinem dem Schaftteil 3 gegenüberliegenden Ende einen verbreiterten Rand bzw. Kragen 6 auf, so daß eine ebene Stirnfläche 7 gebildet ist, die einen Durchmesser aufweist, der größer ist als der Durchmesser des kugelsegmentförmigen Abschnittes des Kopfes. In der Stirnfläche 7 ist ferner eine Ausnehmung zum Ineingriffbringen mit einem Eindrehwerkzeug gebildet.

Das Aufnahmeteil 5 ist im wesentlichen zylindersymmetrisch ausgebildet und weist an seinem einen Ende eine axialsymmetrisch ausgerichtete erste Bohrung 10 auf, deren Durchmesser größer ist als der des Gewindeabschnitts des Schaftes 3 und kleiner als der Kugeldurchmesser des kugelsegmentförmigen Abschnitts des Kopfs 4. Ferner weist es eine koaxiale zweite Bohrung 11 auf, die an dem der ersten Bohrung 10 gegenüberliegenden Ende offen ist und deren Durchmesser so groß ist, daß das Schraubenelement 2 lurch das offenen Ende mit seinem Gewindeabschnitt durch die erste Bohrung 10 hindurch und mit dem kugelsegmentförmigen Abschnitt des Kopfs 4 bis zum Grund der zweiten Bohrung führbar ist. Angrenzend an die erste Bohrung 10 ist in dem Aufnahmeteil ein hohlkugelsegmentförmiger Abschnitt 12 vorgesehen, dessen Radius im wesentlichen gleich dem Radius des kugelsegmentförmigen Abschnitts des Kopfes 4 ist. Ferner weist das Aufnahmeteil eine sich von dem offenen Ende gegen die erste Bohrung 10 hin erstreckende U-förmige Ausnehmung 13 auf, deren Grund zur ersten Bohrung 10 hin gerichtet ist und durch die zwei freie Schenkel 14 gebildet sind, von denen in den Figuren nur einer dargestellt ist. Angrenzend an das freie Ende der Schenkel 14 ist in dem Aufnahmeteil ein Innengewinde 15 ausgebildet. Die Breite der U-förmigen Ausnehmung 13 ist geringfügig größer als der Durchmesser eines darin aufzunehmenden Stabes 100, welcher mehrere solcher Polyaxialschrauben verbindet. Die Tiefe der U-förmigen Ausnehmung ist so bemessen, daß bei eingelegtem Stab eine Fixierschraube 16 zwischen die Schenkel einschraubbar ist.

Der kugelsegmentförmige Abschnitt 12 des Aufnahmeteils ist vorzugsweise glatt poliert oder mit einem die Gleitfähigkeit erhöhenden Material beschichtet, so daß der Kopf 4 leicht in dem kugelsegmentförmigen Abschnitt des Aufnahmeteils schwenkbar ist. Alternativ oder zusätzlich ist der Kopf 4 glatt poliert oder beschichtet.

Zwischen dem eingelegten Stab 100 und dem Kopf 4 des Schraubenelements ist ein Druckelement 20 vorgesehen. Das Druckelement 20 ist zylinderförmig ausgebildet und hat einen Durchmesser, der kleiner ist, als der Innendurchmesser der zweiten Bohrung 11 des Aufnahmeteils und der bevorzugt gleich dem Durchmesser der Stirnfläche 7 des Kopfs ist. Die axiale Länge des Druckelements 20 ist etwas größer oder gleich dem Abstand, den die Stirnfläche 7 des Kopfs 4 in eingesetztem Zustand von dem Grund der U-förmigen Ausnehmung 13 hat. Das Druckelement ist elastisch ausgebildet, in dem dargestellten Ausführungsbeispiel ist es aus einem Elastomer gebildet.

Zwischen dem Druckelement 20 und dem eingelegten Stab 100 ist ferner eine das Druckelement auf der dem Stab zugewandten Seite bedeckende Kappe 21 vorgesehen, die aus einem starren Material, beispielsweise aus Kunststoff oder einem körperverträglichen Metall ausgebildet'ist. Der Außendurchmesser der Kappe 21 ist so bemessen, daß die Kappe in der zweiten Bohrung des Aufnahmeteils gleitend verschiebbar ist, und der Innendurchmesser der Kappe entspricht im wesentlichen dem Außendurchmesser des Druckelements 20 wenn sich dieses in einem unbelasteten Zustand befindet. Die Kappe übergreift das Druckelement gerade um soviel, daß sich das Druckelement bei Belastung in radialer Richtung ausdehnen kann.

Fig. 1 zeigt den unbelasteten Zustand, in dem das Schraubenelement 2, das Druckelement 20 und die Kappe 21 in das Aufnahmeteil eingesetzt sind und der Stab 100 in die U-förmige Ausnehmung 13 eingelegt ist, aber die Innenschraube noch nicht festgezogen ist. In diesem Zustand befindet sich die dem Druckelement 20 abgewandte Seite 22 der Kappe 21 an einer geringfügig höheren Position als der Grund der U-förmigen Ausnehmung 13, so daß der Stab mit seiner Unterseite auf der Oberfläche 22 der Kappe aufliegt und somit ein Spalt 23 zwischen der Unterseite des Stabs und dem Grund der U-förmigen Ausnehmung 13 gebildet ist.

Im Betrieb wird, wie in Fig. 1 gezeigt ist, zuerst das Schraubenelement 2 in das Aufnahmeteil 5 vom offenen Ende desselben her eingeführt bis der Kopf an dem kugelsegmentförmigen Abschnitt 12 des Aufnahmeteils anliegt. Dann wird das Schraubenelement in den Wirbel eingeschraubt. Anschließend wird das Druckelement 20 zusammen mit der aufgesetzten Kappe 21 in das Aufnahmeteil eingesetzt, das Aufnahmeteil ausgerichtet und der Stab eingelegt. Zuletzt wird die Innenschraube 16 in das Aufnahmeteil eingeschraubt.

Wie in Fig. 2 dargestellt ist, wird die Innenschraube soweit eingeschraubt, bis sie den Stab gegen den Grund der U-förmigen Ausnehmung drückt und diesen somit fixiert. Der Stab drückt dabei auf die Kappe 21, die zur gleichmäßigen Verteilung der von dem Stab ausgeübten Druckkraft auf die gesamte Oberfläche des Druckelements dient. Aufgrund der Elastizität des Druckelements wird dieses über die Kraft, die der Stab ausübt vorkomprimiert.Das Druckelement nimmt dabei eine in Fig. 2 gezeigte in radialer Richtung nach außen gewölbte Form an. In dem in Fig. 2 gezeigten Zustand befindet sich das Druckelement 20 unter Vorspannung gegenüber dem Schraubenkopf 4 und drückt aufgrund der rücktreibenden Kraft mit seiner Unterseite gleichmäßig auf die Stirnfläche 7 des Kopfs. Damit wird der Kopf gegen den kugelsegmentförmigen Abschnitt 12 des Aufnahmeteils gedrückt.

Durch eine Eigenbewegung der Wirbelsäule wird das in den Wirbelkörper eingeschraubte Schraubenelement 2 aus seiner Ruhelage herausbewegt. Bei einer Bewegung des Wirbels zum Stab hin unter einem Winkel von 90° zur Stabachse kommt es zu einer gleichmäßigen Kompression des Druckelements, der Winkel des Schafts zum Aufnahmeteil ändert sich dabei nicht. Bei einer Bewegung des Wirbels unter einem anderen Winkel als 90° zur Stabachse, wie in Fig. 3 gezeigt ist, kommt es zu einem Schwenken des Kopfes, der leicht in dem kugelsegmentförmigen Abschnitt 12 des Aufnahmeteils gleitet. Dadurch übt die Stirnfläche 7 des Schraubenkopfs auf einer Seite eine Kompressionskraft auf das Druckelement aus, die dieses einseitig in der Nähe des Randes komprimiert. An der gegenüberliegenden Seite dagegen expandiert das unter Vorspannung stehende Druckelement aufgrund des Nachlassens des Drucks. Somit bleibt das Druckelement immer in Kontakt mit dem Schraubenkopf.

Durch die Komprimierung entsteht aufgrund der Elastizität des Druckelements eine rücktreibende Kraft auf den Schraubenkopf, Damit wird eine Bewegung des Wirbels zurück in seine von dem Chirurgen positionierte Ursprungstellung unterstützt.

Durch die Wahl eines Elastomermaterials für das Druckelement mit einer gewünschten Komprimierbarkeit kann eine Bewegungsbegrenzung des Wirbels eingestellt werden. Ist das Material nur wenig komprimierbar, erlaubt die Vorrichtung nur eine geringe Auslenkung aus der Ruhelage. Ist das Material stark komprimierbar, sind größere Schwenkbereiche möglich. Als Elastomermaterial sind körperverträgliche Elastomere einsetzbar, z. B. Polyurethane oder Polysiloxane.

Der Schwenkbereich ist auch oder zusätzlich einstellbar durch die Wahl des Durchmessers des Kragens 6 des Schraubenkopfs relativ zu dem Durchmesser der zweiten Bohrung des Aufnahmeteils. Wenn der Kragen 6 in geschwenkter Stellung des Schraubenelements 2 an der Wand des Aufnahmeteils anstößt, ist kein weiteres Verschwenken mehr möglich.

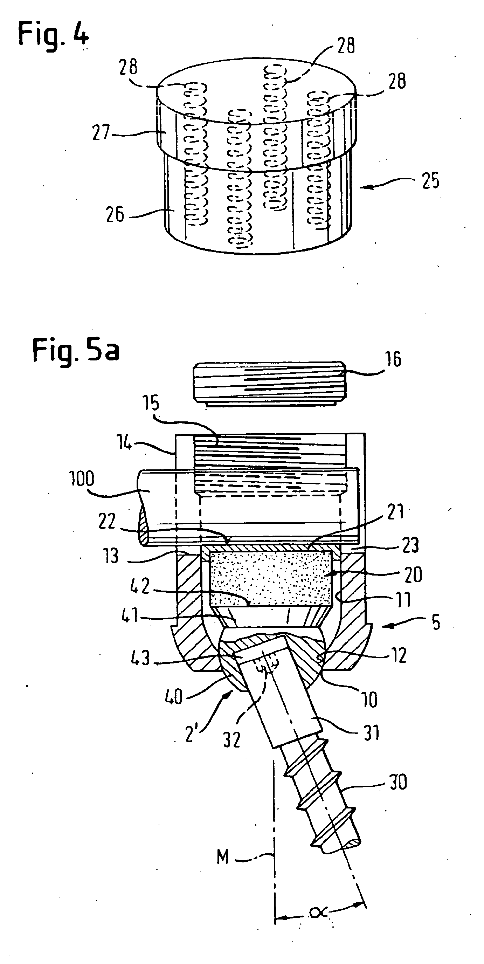

Fig. 4 zeigt ein Druckelement 25 gemäß einer zweiten Ausführungsform. Das Druckelement 25 weist ein dosenförmig ausgebildetes Gehäuse auf, bestehend aus einem Unterteil 26 und einem Oberteil 27, welches das Unterteil wie ein Deckel verschließt. Im Inneren sind wenigstens zwei einander gegenüberliegende, vorzugsweise aber vier oder mehr in Umfangsrichtung gleichmäßig voneinander beabstandete Spiralfedern 28 angeordnet, die mit ihrem einen Ende mit dem Unterteil und mit ihrem anderen Ende mit dem Oberteil verbunden sind. Die Spiralfedern 28 sind in der Nähe der Gehäusewand angeordnet, da, wie aus Fig. 3 ersichtlich ist, die Kompressionskräfte am Rand des Druckelements größer sind, als in der Mitte. Die Stärke der Spiralfedern ist so gewählt, daß eine gewünschte oder erforderliche Kompression über den Schraubenkopf erzielbar ist.

Wenn die Verankerungsvorrichtung das Druckelement gemäß der zweiten Ausführungsform aufweist, ist die bei der ersten Ausführungsform beschriebene Kappe 21 nicht erforderlich, da die Oberseite des Druckelements aus einem starren Material besteht.

Der Betrieb erfolgt wie bei der ersten Ausführungsform. Um ein seitliches Verschieben des Druckelements 25 in dem Aufnahmeteil zu verhindern, ist in einer weiteren Abwandlung der Durchmesser des Druckelements nur geringfügig kleiner als der Durchmesser der zweiten Bohrung 11 des Aufnahmeteils.

In einer weiteren Ausführungsform ist das Druckelement selbst als Spiralfeder ausgebildet. Der Durchmesser der Spiralfeder entspricht dann dem Durchmesser des dosenförmigen Gehäuses der zweiten Ausführungsform. Auch andere Federarten sind möglich, z.B. eine oder mehrere Tellerfedern.

In einer weiteren Ausführungsform sind in dem aus einem Elastomer gebildeten Druckelement 20 der ersten Ausführungsform Spiralfedern in einer Anordnung gleich oder ähnlich der gemäß Fig. 4 enthalten, die bei der Herstellung des Druckelements miteingegossen werden. Diese dienen dann zur Unterstützung der elastischen Eigenschaften des Elastomers.

In einer Abwandlung der ersten Ausführungsform sind das Druckelement 20 und die Kappe 21 in dem Aufnahmeteil 5 vormontiert und durch Herausfallen gesichert, z.B. durch in dem Aufnahmeteil vorgesehene Kröpfbohrungen und damit korrespondierende Senkbohrungen in dem Druckelement. In diesem Fall weisen das Druckelement 20 und die Kappe 21 eine koaxiale zentrale Bohrung auf, die das Hindurchführen eines Schraubwerkzeugs ermöglicht, um das Schraubenlement 2 in den Knochen einzuschrauben. Die Bohrung kann auch vorgesehen sein, wenn das Druckelement und die Kappe nicht vormontiert sind.

In einer weiteren Ausführungsform ist das Druckelement so ausgebildet, daß die der Kappe 21 und/oder der Stirnfläche 7 des Kopfs zugewandte Fläche zum Inneren des Druckelements hin konkav gewölbt ist. Dies im Zusammenwirken mit der ebenen Fläche der Kappe und/oder des Schraubenkopfs eine Erhöhung der Druckelastizität am Rand.

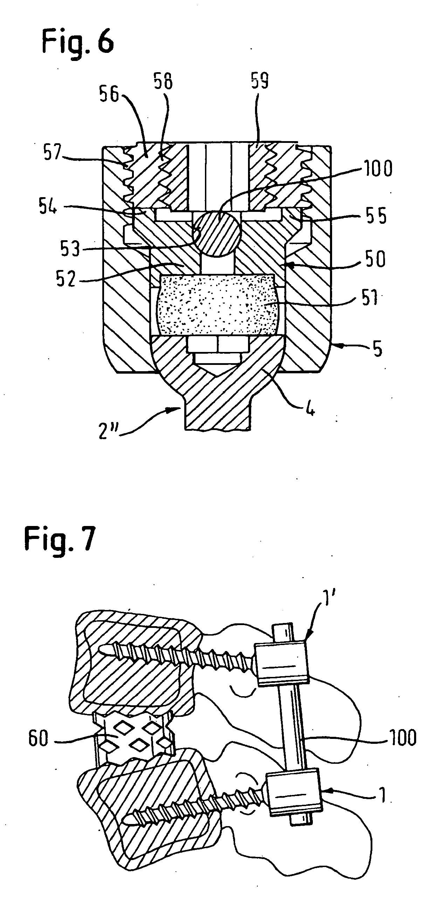

In einer weiteren Abwandlung weist der Kopf den Kragen nicht auf, sondern ist z.B. halbkugelförmig, wie in Fig. 6 dargestellt ist, ausgebildet, so daß die Stirnfläche den größten Durchmesser des Schraubenkopfs aufweist. Entscheidend ist, daß zum Zusammenwirken mit dem Druckelement eine ausreichend große Stirnfläche vorhanden ist, die die Kraftübertragung am Rand gewährleistet.

In einer in Fig. 5a gezeigten zweiten Ausführungsform der Verankerungsvorrichtung liegt die Ruhestellung des Schraubenelements 2' relativ zu dem Aufnahmeteil 5 bei einem Winkel α zur Mittenachse M des Aufnahmeteils, der verschieden von 0° ist. In diesem Fall ist das Schraubenelement 2' zweiteilig ausgebildet. Es umfaßt einen Kopf 40 und einen damit verbindbaren Gewindeschaft 30. Der Gewindeschaft 30 weist an seinem einen Ende einen gewindefreien Abschnitt 31 auf. An diesem Ende ist ferner stirnseitig eine Ausnehmung 32 zum Ineingriffbringen mit einem Schraubwerkzeug vorgesehen. Der Kopf 40 beinhaltet wie bei dem Schraubenelement der ersten Ausführungsform einen kugelsegmentförmigen Abschnitt und einen sich an der dem Schaft abgewandten Seite daran anschließenden Kragen auf 41, der eine Stirnfläche 42 zum Zusammenwirken mit dem Druckelement hat. An der der Stirnfläche abgewandten Seite weist'der Kopf 40 eine unter einem vorgegebenen Winkel α zur Symmetrieachse des Kopfs verlaufende Bohrung 43 auf. Der Durchmesser der Bohrung ist gleich dem Außendurchmesser des gewindefreien Abschnitts 31 des Schafts 30, so daß der Schaft in die Bohrung reibschlüssig einschiebbar ist. Zum Verbessern der Klemmwirkung auf den eingeschobenen Schaft kann der Kopf nicht dargestellte Schlitze in seiner Wand aufweisen, so daß die Bohrung einen federnden Rand besitzt.

Alternativ ist in der Bohrung 43 ein Innengewinde und auf dem Schaftabschnitt 31 ein dazu passendes Außengewinde vorgesehen, so daß der Schaft in den Kopf einschraubbar ist.

Die übrigen Teile des Verankerungsvorrichtung sind wie bei der Ausführungsform nach den Figuren 1 bis 3 bzw. deren Abwandlungen.

Im Betrieb wird zuerst der Schaft 30 in den Wirbel eingeschraubt. Dann wird das Aufnahmeteil 5 mit seiner ersten Bohrung 10 auf den Schaft 30 bzw. auf den herausstehenden gewindefreien Abschnitt 31 schräg aufgesetzt und dann der Kopf 40 mit dem Druckelement und gegebenenfalls der Kappe in vorkomprimiertem Zustand mit einem geeigneten Werkzeug in das Aufnahmeteil eingeführt und mit der Bohrung 43 auf den Schaft aufgeschoben. Die Längsachse des Schaftes 30 weist somit zu der Mittenachse des Kopfes 40 und damit zur Mittenachse des Aufnahmeteils 5 einen vorgegebenen Winkel α auf. Anschließend wird dann der Stab eingelegt und schließlich alles über die Innenschraube 16 fixiert, wie bei der ersten Ausführungsform. Wie bei der ersten Ausführungsform bewirkt bei einer Bewegung des Wirbels die rücktreibende Kraft, daß der Kopf wieder in seine Ruhelage zurückgedrängt wird. Obwohl die Winkelstellung durch den Winkel der Bohrung 43 vorgegeben ist, kann der Chirurg durch Drehen des Aufnahmeteils um seine Achse oder ein geringfügiges Schwenken des Kopfes noch eine Justierung vornehmen.

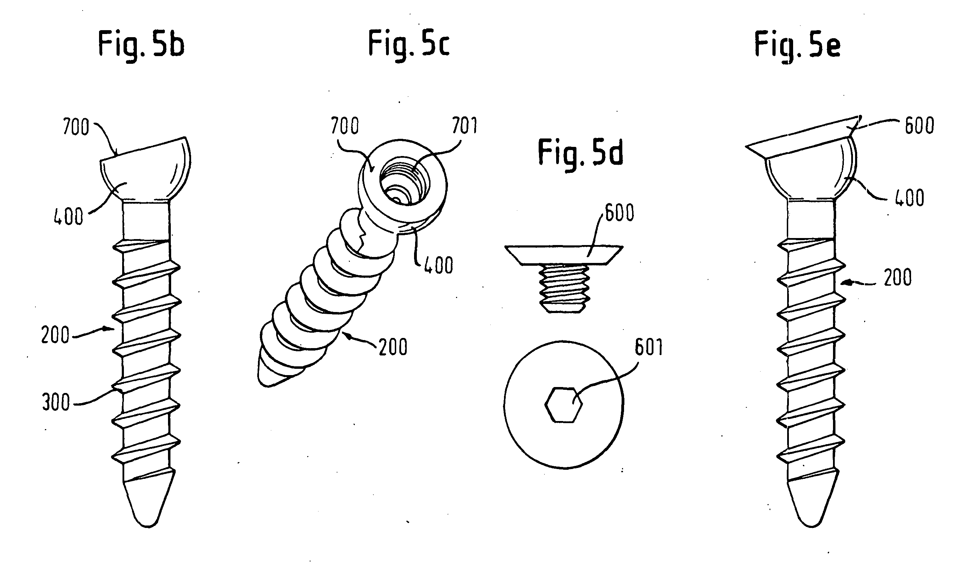

In einer weiteren Abwandlung der zweiten Ausführungsform ist der Kopf einstückig mit dem Schaft ausgebildet, die Stirnfläche des Kopfes, die die Auflagefläche für das elastische Druckelement bildet jedoch einen Winkel zur Schaftachse. In den Figuren 5b bis 5e sind die Herstellungsschritte eines solchen Schraubenelements 200 gezeigt. Das Schraubenelement 200 weist einen Gewindeschaft 300 und einen damit einstückig verbundenen kugelsegmentförmigen Kopf 400 auf. Dieser wird, wie in Fig. 5b gezeigt ist, so abgefräst, daß die Stirnfläche 700 einen vorgegebenen Winkel zur Schaftachse aufweist. Anschließend wird, wie in Fig. 5c gezeigt ist, eine Bohrung 701 mit Innengewinde erzeugt, die senkrecht zur Stirnfläche 700 verläuft. In diese Bohrung wird, wie in Fig. 5d gezeigt ist, eine Schraube 600 mit einem kragenförmigen Kopf und mit einer Ausnehmung 601 zum späteren Einschrauben in den Knochen in den Kopf eingeschraubt, wobei der Durchmesser des Kragens größer ist, als der Durchmesser des Kopfs 400. Das in Fig. 5e gezeigte fertige Schraubenelement 200 wird sodann in das Aufnahmeteil eingesetzt.

Im Betrieb wird das Schraubenelement 200 in das Aufnahmeteil eingesetzt und anschließend in den Knochen eingeschraubt. Durch die unter dem vorgegebenen Winkel zur Schaftachse verlaufende Fläche 700 weist das Schraubenelement 200 in Ruhestellung den vorgegebenen Winkel zu dem Aufnahmeteil auf. Der Einsatz des Druckelements und des Stabs erfolgt wie zuvor beschrieben.

Der Kragen kann auch einstückig mit dem Schraubenelement ausgebildet sein.

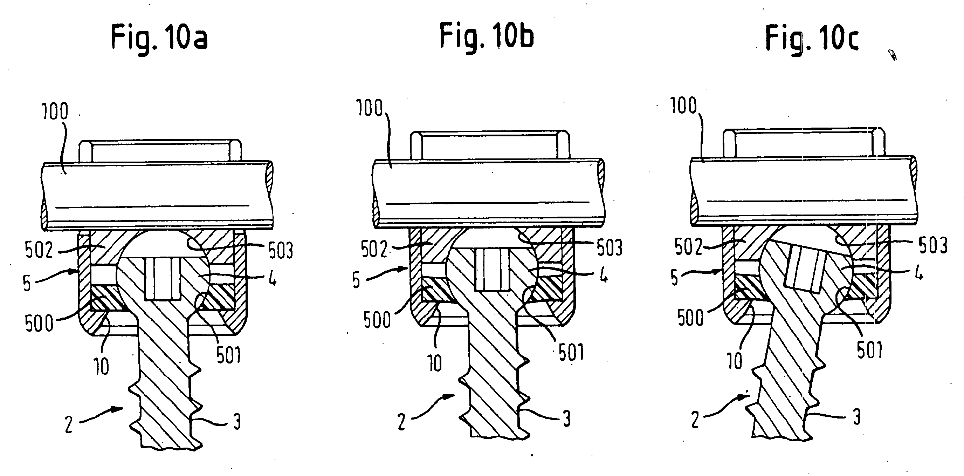

In einer weiteren, in den Figuren 10a-10c gezeigten Abwandlung der zweiten Ausführungsform weist nicht das Aufnahmeteil 5 selbst den kugelsegmentförmigen Abschnitt auf, an dem der Kopf anliegt, sondern es ist in dem Aufnahmeteil ein Einsatzelement 500 aus einem Elastomer vorgesehen, das mit einer zylindrische Außenwand ausgebildet ist und den kugelsegmentförmigen Abschnitt des Kopfs von der ersten Bohrung 10 beginnend seitlich umschließt, oder das, wie in Fig. 10a gezeigt ist, ringförmig ausgebildet ist und nur eine Anlagefläche 501 für den Schraubenkopf aufweist. Es ist ferner ein starres Druckelement 502 vorgesehen, das auf den Kopf 4 drückt. Das Druckelement 502 weist eine kugelsegmentförmige Ausnehmung auf, das in den Schraubenkopf paßt. Im Betrieb wird nach Justieren von Schraube 2 und Aufnahmeteil 5 zueinander der Schraubenkopf 4 über die Fixierschraube 16 und das Druckelement 502 in einer gewünschten Winkelstellung gegen das Einsatzelement gedrückt, welches dabei, weil es leicht zusammengedrückt wird, unter Vorspannung steht, was in Fig. 10b gezeigt ist. Diese Stellung bildet die Ruhestellung. Bei Bewegung des Wirbels drückt der Schraubenkopf gegen das Einsatzelement 500, so daß sich diesen an der Stelle verformt und dabei eine Auslenkung der Schraube aus der Ruhestellung ermöglicht, was in Fig. 10c gezeigt ist. Gleichzeitig drängt die entstehende rücktreibende Kraft den Kopf wieder in seine Ruhestellung. In dieser Ausführungsform ist die gewünschte Winkelstellung von dem Schraubenschaft zu dem Aufnahmeteil, die die Ruhestellung bilden soll, frei einstellbar.

In einer weiteren Abwandlung ist das Druckelement keilförmig ausgebildet.

In einer dritten in Fig. 6 gezeigten Ausführungsform ist die Verankerungsvorrichtung so ausgebildet, daß der Kopf und der Stab unabhängig belastet werden können. Hierzu weist im Unterschied zur ersten Ausführungsform das Druckelement 50 einen ersten, elastischen Abschnitt 51 und angrenzend daran einen zweiten starren Abschnitt 52 auf, die miteinander fest verbunden sind, der eine U-förmige Ausnehmung 53 hat, deren Abmessungen so bemessen sind, daß darin der Stab 100 einlegbar ist. Die in Richtung der Zylinderachse gesehene Tiefe der U-förmigen Ausnehmung des Druckelements ist größer als der Durchmesser des Stabs. Dadurch stehen die durch die U-förmige Ausnehmung gebildeten freien Schenkel 54, 55 bei eingelegtem Stab über diesen hinaus. Es ist ferner ein mutter- bzw. muffenartiges Verschlußelement 56 vorgesehen, das ein mit dem Innengewinde des Aufnameteils zusammenwirkendes Außengewinde 57 und ein Innengewinde 58 aufweist. In das Verschlußelement 56 ist eine Innenschraube 59 vorgesehen.

Im Betrieb wird über das Verschlußelement 56 wenn eine Kraft auf die Schenkel des starren Druckelementsteils 52 und des elastischen Druckelementteils 51 damit auf den Schraubenkopf aufgebracht. Der Stab wird unabhängig davon über die Innenschraube 59 fixiert. Wenn es gewünscht ist, kann der Stab auch beweglich gehalten werden, In diesem Fall wird die Innenschraube nur soweit eingeschraubt, daß der Stab noch in der U-förmigen Ausnehmung des Aufnahmeteils gleiten kann.

Das Druckelement 50 ist in der gezeigten Ausführungsform, bei der der Abschnitt 51 und 52 fest miteinander verbunden sind, einteilig ausgebildet. In einer Abwandlung ist es zweiteilig ausgebildet und besteht dann aus einem elastischen Teil 51 gemäß der Ausführungsform nach Fig. 1 und einem nichtelastischen Teil 52, der die U-förmige Ausnehmung aufweist.

Fig. 7 zeigt eine dynamische Stabilisierungseinrichtung für Wirbel nach einer ersten Ausführungsform. Diese ist insbesondere in einem Fall anwendbar, bei dem eine Bandscheibe oder ein Wirbel entfernt ist und durch ein starres Fusionselement 60, z.B. ein rohrförmiges Implantat mit Öffnungen in der Wandung und z.B. mit Knochenmasse aufgefüllt zum Erlauben des Einwachsens von Knochen, ersetzt ist.

Die Stabilisierungseinrichtung weist im posterioren Bereich zwei dynamische Knochenverankerungsvorrichtungen 1, 1' auf, die über einen starren Stab 100 miteinander verbunden sind. Jede der Knochenverankerungsvorrichtungen 1, 1' ist nach einer der zuvor beschriebenen Ausführungsformen ausgebildet. Durch die elastische Ausbildung des Druckelements und die damit verbundene Dämpfungswirkung kommt es zu einer intermittierenden begrenzten Belastung des vorderen Fusionselements 60, was eine Beschleunigung des Einwachsens von Knochen in in und um das Fusionselement zur Folge hat. Damit wird der Heilungsprozeß beschleunigt.

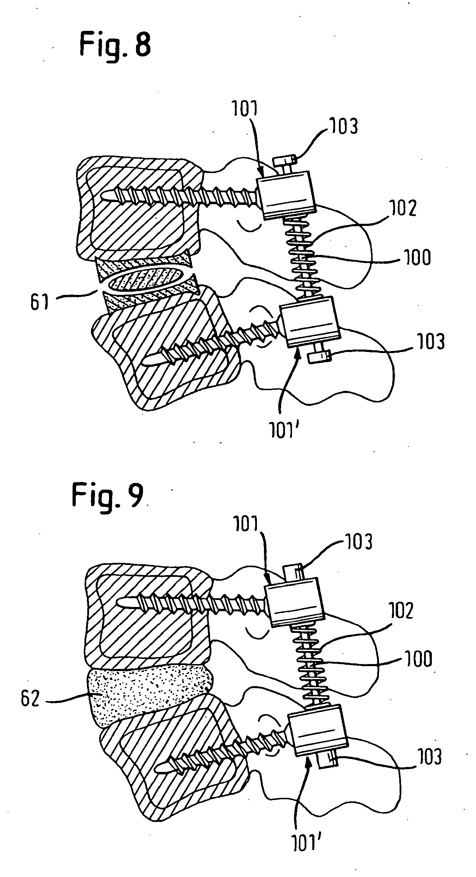

Fig. 8 zeigt eine dynamische Stabilisierungseinrichtung für Wirbel nach einer zweiten Ausführungsform. Diese ist insbesondere in einem Fall anwendbar, in dem eine Bandscheibe entfernt wurde und durch eine Bandscheibenprothese 61 ersetzt wurde. Die Stabilisierungseinrichtung umfaßt zwei dynamische Verankerungsvorrichtung 101, 101', die über einen starren Stab 100 miteinander verbunden sind. Wenigstens eine der Verankerungsvorrichtungen 101, 101' ist nach der in Fig. 6 gezeigten Ausführungsform ausgebildet, die sich dadurch auszeichnet, daß über das Druckelement 50 Druck auf den Schraubenkopf so ausgeübt wird, der Stab aber in axialer Richtung verschiebbar bleibt. Die Stabilisierungseinrichtung umfaßt ferner ein zwischen den beiden Verankerungsvorrichtungen auf dem Stab vorgesehenes Federelement 102, sowie wenigstens einen Anschlag 103 an dem Stab, der an der dem Federelement 102 gegenüberliegenden Seite derjenigen Verankerungsvorrichtung angeordnet ist, in welcher der Stab 100 gleitend gehalten ist. In dem in Fig. 8 gezeigten Beispiel sind zwei solche Anschläge vorgesehen und der Stab ist in beiden Verankerungsvorrichtungen 101, 101' gleitend gehalten. Das Federelement 102 ist unter Vorspannung zwischen den Verankerungsvorrichtungen eingebracht und wirkt somit als Extensionsfeder.

Im Betrieb ermöglicht die Kombination von posteriorer Längsfederung des Stabes 100 und der polyaxialen Dämpfung der Verankerungsvorrichtungen 101, 101' eine Bewegungskontrolle und eine Entlastung der Bandscheibenprothese. Die Stabilisierungseinrichtung ist für jede künstliche Bandscheibenkonstruktion anwendbar.

Fig. 9 zeigt eine weitere Anwendung der in Fig. 8 dargestellten Stabilisierungseinrichtung, die im posterioren Bereich den Extensionsfederstab und die dynamischen Verankerungsvorrichtungen in Form der zuvor beschriebenen polyaxialen Dämpfungsschrauben umfaßt. Bei diesem Anwendungsfall ist die menschliche Bandscheibe insoweit beschädigt, daß sie sich unter Entlastung wieder erholt. Die Stabilisierungseinrichtung entlastet dabei die menschliche Bandscheibe und begrenzt gleichzeitig den Bewegungsumfang, so daß extreme, die Bandscheibe weiter schädigende Bewegungen nicht auftreten können und sich die Bandscheibe in der Ruhephase, z.B. nachts oder im Liegen, erholen kann.

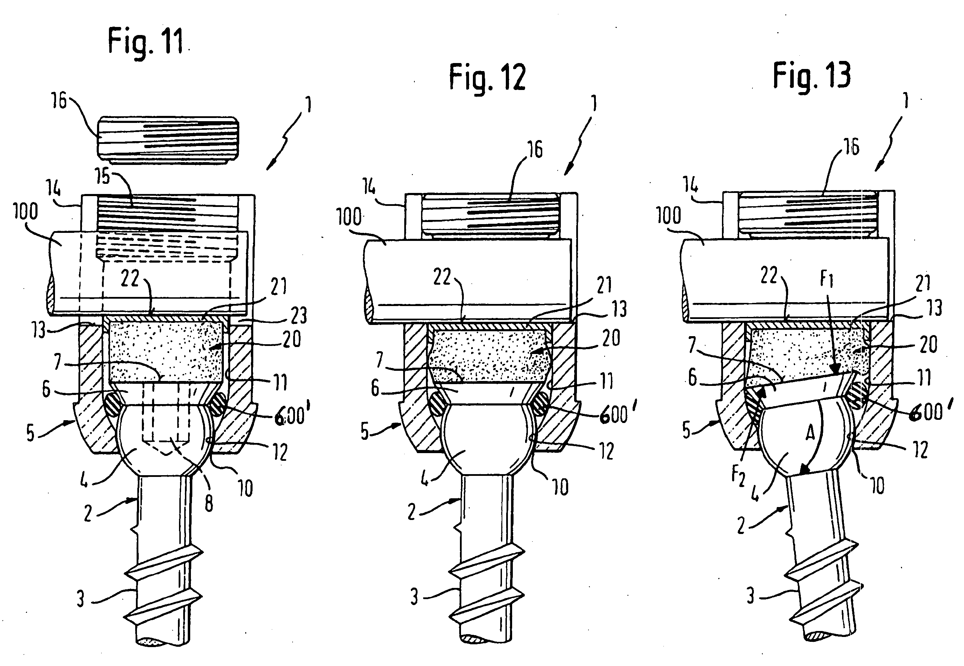

In den Figuren 11 bis 13 ist eine weitere Ausführungsform der Erfindung beschrieben. Elemente, die den Elementen der in den Figuren 1 bis 3 gezeigten Ausführungs form entsprechen, sind mit denselben Bezugszeichen versehen. Gemäß Fig. 11 ist bei dieser Ausführungsform zusätzlich zu dem zwischen dem Stab und dem Kopf angeordneten elastischen Druckelement 20 ein zweites elastisches Druckelement 600' vorgesehen. Das zweite Druckelement 600' ist ringförmig ausgebildet und so angeordnet, daß es den Schraubenkopf an der Stelle umfasst, an der der Kragen 6 an den kugelsegmentförmigen Abschnitt angrenzt. In dem gezeigten Ausführungsbeispiel ist das zweite Druckelement 600' als O-Ring ausgebildet. Der Durchmesser des Druckelements und sein Ringdurchmesser sind so bemessen, dass in dem in Fig. 11 dargestellten in das Aufnahmeteil 5 eingesetzten unbelasteten Zustand der Ring die Außenseite des Kopfes und die Innenwand des Aufnahmeteils berührt. Das zweite Druckelement 600' ist zweckmäßigerweise beim Einsetzen des Schraubenelements in das Aufnahmeteil schon auf den Schraubenkopf aufgesetzt.

Im Betrieb verformt sich das Druckelement 600' in dem in Fig. 12 dargestellten belasteten Zustand, in dem der Stab fixiert ist. Durch die bei der Verformung auftretende Komprimierung wird eine Vorspannung in Richtung des Schraubenkopfes erzeugt. Gemäß Fig. 13 erzeugt bei einer Bewegung des Schraubenelements 2 aus seiner Ruhelage heraus das erste Druckelement 20 eine auf die Stirnfläche 7 des Schraubenkopfs von oben wirkende rücktreibende Kraft, die durch den Pfeil F₁ dargestellt ist, während das zweite Druckelement 600', eine von seitlich unterhalb der Stirnfläche 7 und zu der Kraft F₁ diagonal versetzt wirkende rücktreibende Kraft auf den Schraubenkopf erzeugt, die durch den Pfeil F₂ gekennzeichnet ist. Die auf den Schraubenkopf wirkenden Rückstellkräfte, die diesen, wie durch den Pfeil A in Fig. 13 gezeigt ist, wieder in die Ausgangslage bringen, werden somit durch das zweite Druckelement 600' erhöht. Ferner bewirkt das zweite Druckelement 600' eine Verminderung des Verschleißes des ersten Druckelements 20.

Durch Wahl der Materialien für das erste Druckelement 20 und das zweite Druckelement 600', die verschieden voneinander sein können, kann eine gewünschte Einstellung der Dämpfung erfolgen. Zum Beispiel ist das erste Druckelement aus einem härteren bzw. schwerer komprimierbaren Material gebildet als das zweite Druckelement.

In einer Abwandlung dieser Ausführungsform ist das zweite Druckelement 600' nicht als O-Ring ausgebildet, sondern als ein Formring.

Die Erfindung ist nicht auf die oben beschriebenen Ausführungsformen beschränkt. Kombinationen von Elementen der einzelnen Ausführungsformen mit Elementen anderer Ausführungsformen sind möglich. Insbesondere ist auch die Erfindung nicht auf eine Polyaxialschraube beschränkt, sondern als Verankerungselement kann auch ein Haken anstelle des Gewindeschafts vorgesehen sein. Ferner kann das Aufnahmeteil auch so ausgebildet sein, daß die Schraube von unten in das Aufnahmeteil einführbar ist. In diesem Fall ist ein Formring als Widerlager für den Schraubenkopf vorgesehen.

## Patentansprüche

1. Dynamische Verankerungsvorrichtung mit einem Element (2, 2', 2", 200) mit einem Schaft (3, 30, 300) zur Verankerung in einem Knochen oder einem Wirbel und mit einem mit dem Schaft (3, 30) verbundenen Kopf (4, 40, 400) sowie einem Aufnahmeteil (5) dafür,
wobei das Aufnahmeteil eine U-förmige Ausnehmung zum Einlegen eines Stabs aufweist und wobei eine Fixierschraube (16, 56, 59) zum Fixieren des Stabs in der Ausnehmung vorgesehen ist, und
mit einem auf den Kopf einwirkenden Druckelement (20, 25, 50)
**gekennzeichnet dadurch daß** das Druckelement einen elastischen Abschnitt aufweist, dessen Durchmesser kleiner ist, als der Innendurchmesser des Aufnahmeteils und der in fixiertem Zustand des Stabs vorkomprimiert ist, derart, dass er bei einer Bewegung des Elements (2, 2', 2 " , 200), bei der der Kopf in dem Aufnahmeteil geschwenkt wird, eine rücktreibende Kraft auf den Kopf ausübt.

2. Dynamische Verankerungsvorrichtung nach Anspruch 1, **dadurch gekennzeichnet, daß** das Druckelement (20, 25, 50) auf die dem Schaft (3 , 30) abgewandten Seite des Kopfs (4 , 40) einwirkt.

3. Dynamische Verankerungsvorrichtung nach Anspruch 1 oder 2, **dadurch gekennzeichnet, daß** das Druckelement (20, 51) aus einem Elastomer ausgebildet ist.

4. Dynamische Verankerungsvorrichtung nach Anspruch 1 oder 2, **dadurch gekennzeichnet, daß** das Druckelement (25) wenigstens ein Federelement (28) aufweist.

5. Dynamische Verankerungsvorrichtung nach einem der Ansprüche 1 bis 4, **dadurch gekennzeichnet, daß** der Kopf (4, 40) an seiner dem Schaft (3, 30) abgewandten Seite eine ebene Fläche (7, 42) aufweist und das Drückelement (20, 25, 50) eine mit dieser zusammenwirkende ebene Fläche aufweist.

6. Dynamische Verankerungsvorrichtung nach Anspruch 5, **dadurch gekennzeichnet, daß** der Kopf (4, 40) an seinem dem Schaft abgewandten Ende einen verbreiterten Rand (6, 41) aufweist.

7. Dynamische Verankerungsvorrichtung nach einem der Ansprüche 1 bis 6, **dadurch gekennzeichnet, daß** das Aufnahmeteil (5) eine Anlagefläche (12) für den Kopf (4, 40) aufweist und daß die Anlagefläche und/oder der Kopf zur Reduzierung der Reibung poliert oder beschichtet sind.

8. Dynamische Verankerungsvorrichtung nach einem der Ansprüche 1 bis 7, **dadurch gekennzeichnet, daß** an der in dem in das Aufnahmeteil (5) eingesetzten Zustand dem Kopf (4, 40) abgewandten Seite des Druckelements (20) ein starres Zwischenelement (21) vorgesehen ist.

9. Dynamische Verankerungsvorrichtung nach einem der Ansprüche 1 bis 8, **dadurch gekennzeichnet, daß** der Kopf (40) und der Schaft (30) als separate Teile ausgebildet sind und der Schaft (30) unter einem vorgegebenen Winkel α zur Mittenachse des Kopfes mit dem Kopf verbindbar ist.

10. Dynamische Verankerungsvorrichtung nach einem der Ansprüche 1 bis 9, **dadurch gekennzeichnet, daß** das Druckelement (50) zylindrisch ausgebildet ist und einen ersten, elastischen Abschnitt (51) aufweist und einen daran angrenzenden zweiten starren Abschnitt (52) mit einer U-förmigen Ausnehmung (53) aufweist, durch die zwei freie Schenkel (54, 55) gebildet werden, wobei die Tiefe der U-förmigen Ausnehmung (53) größer ist als der Durchmesser eines in das Aufnahmeteil einzulegenden Stabs (100).

11. Dynamische Verankerungsvorrichtung nach Anspruch 10, **dadurch gekennzeichnet, daß** das Druckelement (50) zweiteilig ausgebildet ist, wobei ein Teil durch den elastischen Abschnitt (51) und das andere Teil durch den starren Abschnitt (52) mit der U-förmigen Ausnehmung gebildet wird.

12. Dynamische Verankerungsvorrichtung nach einem der Ansprüche 1 bis 11, **dadurch gekennzeichnet, daß** das Druckelement (20, 25, 50) ein erstes Druckelement ist und daß ein zweites elastisches Druckelement (600') vorgesehen ist, das den Schraubenkopf ringförmig umgreift.

13. Dynamische Verankerungsvorrichtung nach Anspruch 14, **dadurch gekennzeichnet, daß** das zweite Druckelement als O-Ring oder als Formring ausgebildet ist.

14. Dynamische Verankerungsvorrichtung nach einem der Ansprüche 1 bis 13, **dadurch gekennzeichnet, dass** das Druckelement (20, 25, 50) zwischen Kopf und Stab angeordnet ist und so ausgebildet ist, daß es durch den Druck des eingelegten Stabes vorkomprimiert ist, wenn dieser im Grund der U-förmigen Ausnehmung aufliegt.

15. Dynamische Stabilisierungseinrichtung für Knochen, insbesondere für Wirbel, mit mindestens zwei Verankerungsvorrichtungen (1, 1'; 101, 101'), die mit einem Stab (100) verbunden sind, **dadurch gekennzeichnet, daß** wenigstens eine der Verankerungsvorrichtungen (1, 1'; 101, 101') eine dynamische Verankerungsvorrichtung nach einem der Ansprüche 1 bis 14 ist.

16. Dynamische Stabilisierungseinrichtung nach Anspruch 15, **dadurch gekennzeichnet, daß** zwischen den Verankerungsvorrichtungen (101, 101') auf dem Stab ein Federelement (102) vorgesehen ist.

17. Dynamische Verankerungsvorrichtung mit einem Element (2), mit einem Schaft (3) zur Verankerung in einem Knochen oder einem Wirbel und mit einem mit dem Schaft verbundenen Kopf (4) sowie einem Aufnahmeteil (5) dafür,
wobei das Aufnahmeteil eine U-förmige Ausnehmung zum Einlegen eines Stabs aufweist und wobei eine Fixierschraube (16) zum Fixieren des Stabs in der Ausnehmung vorgesehen ist, mit einem starren Druckelement (502), das auf der der Fixierschraube zugewandten Seite des Kopfs vorgesehen ist und auf den Kopf einwirkt und
mit einem elastischen Einsatzelement (500), das in dem Aufnahmeteil vorgesehen ist und den Kopf seitlich umschließt,
**dadurch gekennzeichnet daß,** das Einsatzelement (500) in fixiertem Zustand des Stabs vorkomprimiert ist, derart, dass es bei einer Bewegung des Elements (2) bei der der Kopf (4) in dem Aufnahmeteil (5) geschwenkt wird, eine rücktreibende Kraft auf den Kopf ausübt.

18. Dynamische Verankerungsvorrichtung nach Anspruch 17, wobei das Einsatzelement (500) aus einem Elastomer gebildet ist.

19. Dynamische Verankerungsvorrichtung nach Anspruch 17 oder 18, wobei das Einsatzelement ringförmig ausgebildet ist.

## Claims

1. Dynamic anchoring device comprising an element (2, 2', 2" , 200) having a shank (3, 30, 300) for anchoring in a bone or a vertebra and a head (4, 40, 400) connected to the shank (3, 30) and comprising a receiving part (5) for receiving said head,
wherein the receiving part has a U-shaped recess for inserting a rod and wherein a fixing screw (16, 56, 59) is provided for fixing the rod in the recess, and
comprising pressure elements (20, 25, 50) acting on the head, **characterized in that** the pressure element has a resilient portion, the diameter of which is smaller than the inside diameter of the receiving part and which is pre-compressed in the fixed state of the rod in such a way that, when there is a movement of the element (2, 2', 2'', 200) in which the head is swivelled in the receiving part, it exerts a return force on the head.

2. Dynamic anchoring device according to Claim 1, **characterized in that** the pressure element (20, 25, 50) acts on the side of the head (4, 40) facing away from the shank.

3. Dynamic anchoring device according to Claim 1 or 2, **characterized in that** the pressure element (20, 51) is formed from an elastomer.

4. Dynamic anchoring device according to Claim 1 or 2, **characterized in that** the pressure element (25) has at least one spring element (28).

5. Dynamic anchoring device according to one of Claims 1 to 4, **characterized in that** the head (4, 40) has a flat surface (7, 42) on its side facing away from the shank (3, 30) and the pressure element (20, 25, 50) has a flat surface cooperating therewith.

6. Dynamic anchoring device according to Claim 5, **characterized in that** the head (4, 40) has a widened periphery (6, 41) at its end facing away from the shank.

7. Dynamic anchoring device according to one of Claims 1 to 6, **characterized in that** the receiving part (5) has a bearing surface (12) for the head (4, 40) and **in that** the bearing surface and/or the head is/are polished or coated to reduce friction.

8. Dynamic anchoring device according to one of Claims 1 to 7, **characterized in that** a rigid intermediate element (21) is provided on the side of the pressure element (21) facing away from the head (4, 40) in the state in which it is inserted in the receiving part (5).

9. Dynamic anchoring device according to one of Claims 1 to 8, **characterized in that** the head (40) and the shank (30) are formed as separate parts and the shank (30) can be connected to the head at a predetermined angle α in relation to the cental axis of the head.

10. Dynamic anchoring device according to one of Claims 1 to 9, **characterized in that** the pressure element (50) is cylindrically formed and has a first, resilient portion (51) and a second, rigid portion (52), which is adjacent to the said first portion and has a U-shaped recess (53), by which two free legs (54, 55) are formed, wherein the depth of the U-shaped recess (53) is greater than the diameter of a rod (100) to be inserted in the receiving part.

11. Dynamic anchoring device according to Claim 10, **characterized in that** the pressure element (50) is formed as two parts, wherein one part is formed by the resilient portion (51) and the other part is formed by the rigid portion (52) with the U-shaped recess.

12. Dynamic anchoring devices according to one of Claims 1 to 11, **characterized in that** the pressure element (20, 25, 50) is a first pressure element and **in that** a second resilient pressure element (600') is provided, engaging around the screw head in an annular manner.

13. Dynamic anchoring device according to Claim 14, **characterized in that** the second pressure element is formed as an O-ring or as a moulded ring:

14. Dynamic anchoring device according to one of Claims 1 to 13, **characterized in that** the pressure element (20, 25, 50) is arranged between the head and the rod and is formed in such a way that it is pre-compressed by the pressure of the inserted rod when the latter is lying on the bottom of the U-shaped recess.

15. Dynamic stabilizing device for bones, in particular for vertebrae, comprising at least two anchoring devices (1, 1'; 101, 101'), which are connected by a rod (100), **characterized in that** at least one of the anchoring devices (1, 1'; 101, 101') is a dynamic anchoring device according to one of Claims 1 to 14.

16. Dynamic stabilizing device according to Claim 15, **characterized in that** a spring element (102) is provided on the rod between the anchoring devices (101, 101').

17. Dynamic anchoring device comprising an element (2) having a shank (3) for anchoring in a bone or a vertebra and a head (4) connected to the shank and comprising a receiving part (5) for receiving said head,
wherein the receiving part has a U-shaped recess for inserting a rod and wherein a fixing screw (16) is provided for fixing the rod in the recess, comprising a rigid pressure element (502), which is provided on the side of the head facing the fixing screw and acts on the head, and
comprising a resilient insert element (500), which is provided in the receiving part and laterally encloses the head,
**characterized in that** the insert element (500) is pre-compressed in the fixed state of the rod in such a way that, when there is a movement of the element (2) in which the head (4) is swivelled in the receiving part (5), it exerts a return force on the head.

18. Dynamic anchoring device according to Claim 17, wherein the insert element (500) is formed from an elastomer.

19. Dynamic anchoring device according to Claim 17 or 18, wherein the insert element is formed in an annular manner.

## Revendications

1. Dispositif d'ancrage dynamique, comprenant un élément (2, 2', 2", 200) avec une tige (3, 30, 300), pour l'ancrage dans un os ou dans une vertèbre, et avec une tête (4, 40, 400), reliée à la tige (3, 30), ainsi qu'une partie de réception (5) pour elle,
la partie de réception présentant un évidement en forme de U, pour l'introduction d'une barre, et une vis de fixation (16, 56, 59) étant prévue pour fixer la barre dans l'évidemment; et
avec un élément de pressage (20, 25, 50) agissant sur la vis,
**caractérisé en ce que** l'élément de pressage présente un tronçon élastique, dont le diamètre est inférieur au diamètre intérieur de la partie de réception et qui est précomprimé lorsque la barre est à l'état fixé, de manière que, en cas d'un déplacement de l'élément (2, 2', 2", 200), pour lequel la tête est pivotée dans la partie de réception, une force de rappel soit exercée sur la tête.

2. Dispositif d'ancrage dynamique selon la revendication 1, **caractérisé en ce que** l'élément de pressage (20, 25, 50) agit sur la face de la tête (4, 40) qui est opposée à la tige (3, 30).

3. Dispositif d'ancrage dynamique selon la revendication 1 ou 2, **caractérisé en ce que** l'élément de pressage (20, 51) est formé d'un élastomère.

4. Dispositif d'ancrage dynamique selon la revendication 1 ou 2, **caractérisé en ce que** l'élément de pressage (25) présente au mois un élément élastique (28).

5. Dispositif d'ancrage dynamique selon l'une des revendications 1 à 4, **caractérisé en ce que**, sur sa face opposée à la tige (3, 30), la tête (4, 40) présente une face (7, 42) plane, et l'élément de pressage (20, 25, 50) présente une face plane, coopérant avec celle-ci.

6. Dispositif d'ancrage dynamique selon la revendication 5, **caractérisé en ce que** la tête (4, 40) présente un bord (6, 41) élargi, sur son extrémité opposée à la tige.

7. Dispositif d'ancrage dynamique selon l'une des revendications 1 à 6, **caractérisé en ce que** la partie de réception (5) présente une face d'appui (12) pour la tête (4, 40), et **en ce que** la face d'appui et/ou la tige est/sont polie(s) ou munie(s) d'un revêtement, dans le but de réduire le frottement.

8. Dispositif d'ancrage dynamique selon l'une des revendications 1 à 7, **caractérisé en ce qu'**un élément intermédiaire (21) rigide est prévu, sur la face de l'élément de pressage (20), opposée à la tête (4, 40), à l'état inséré dans la partie de réception (5).

9. Dispositif d'ancrage dynamique selon l'une des revendications 1 à 8, **caractérisé en ce que** la tête (40) et la tige (30) sont réalisées sous forme de pièces séparées et la tige (30) est susceptible d'être reliée à la tête, sous un angle α prédéterminé par rapport à l'axe médian de la tête.

10. Dispositif d'ancrage dynamique selon l'une des revendications 1 à 9, **caractérisé en ce que** l'élément de pressage (50) est cylindrique et présente une premier tronçon (51) élastique, et un deuxième tronçon (52) rigide, lui étant limitrophe, avec un évidement (53) en forme de U, au moyen duquel sont formées deux branches (54, 55) libres, la profondeur de l'évidement (53) en forme de U étant supérieure au diamètre d'une barre (100) à insérer dans la partie de réception.

11. Dispositif d'ancrage dynamique selon la revendication 10, **caractérisé en ce que** l'élément de pressage (50) est réalisé en deux parties, une partie étant formée par le tronçon (51) élastique et l'autre partie étant formée par le tronçon (52) rigide avec l'évidement en forme de U.

12. Dispositif d'ancrage dynamique selon l'une des revendications 1 à 11, **caractérisé en ce que** l'élément de pressage (20, 25, 50) est un premier élément de pressage, et **en ce qu'**un deuxième élément de pressage (600) est prévu, entourant en forme d'anneau la tête de vis.

13. Dispositif d'ancrage dynamique selon la revendication 14, **caractérisé en ce que** le deuxième élément de pressage est réalisé sous forme d'élément torique ou d'anneau de forme.

14. Dispositif d'ancrage dynamique selon l'une des revendications 1 à 13, **caractérisé en ce que** l'élément de pressage (20, 25, 50) est disposé entre tête et barre et est réalisé de manière à être précomprimé par la pression de la barre insérée, lorsque celle-ci repose au fond de l'évidemment.

15. Dispositif de stabilisation dynamique pour des os, en particulier des vertèbres, comprenant au moins deux dispositifs d'ancrage (1, 1' ; 101, 101'), reliés à une barre, **caractérisé en ce qu'**au moins l'un des dispositifs d'ancrage (1, 1' ; 101, 101') est un dispositif d'ancrage dynamique selon l'une des revendications 1 à 14.

16. Dispositif de stabilisation dynamique selon la revendication 15, **caractérisé en ce qu'**un élément élastique (102) est prévu sur la barre, entre les dispositifs d'ancrage (101, 101').

17. Dispositif d'ancrage dynamique, comprenant un élément (2) avec une tige (3), pour l'ancrage dans un os ou dans une vertèbre, avec une tête (4), reliée à la tige, ainsi qu'une partie de réception (5) pour elle,
la partie de réception présentant un évidement en forme de U, pour l'introduction d'une barre, et une vis de fixation (16) étant prévue pour fixer la barre dans l'évidemment,
avec un élément de pressage (502) rigide, prévu sur la face, tournée vers la vis de fixation, de la tête et agissant sur la tête, et
avec un élément formant insert (500) élastique, prévu dans la partie de réception et entourant latéralement la tête,
**caractérisé en ce que** l'élément formant insert (500), à l'état fixé de la barre, est précomprimé, de manière que, en cas d'un déplacement de l'élément (2), pour lequel la tête (4) est pivotée dans la partie de réception (5), une force de rappel soit exercée sur la tête.

18. Dispositif d'ancrage dynamique selon la revendication 17, **caractérisé en ce que** l'élément formant insert (500) est formé d'un élastomère.

19. Dispositif d'ancrage dynamique selon la revendication 17 ou 18, **caractérisé en ce que** l'élément formant insert est de forme annulaire.
